(19) Europäisches Patentamt
European Patent Office
Office européen des brevets

(11) **EP 3 627 857 A1**

(12) **EUROPEAN PATENT APPLICATION**

(43) Date of publication:
**25.03.2020 Bulletin 2020/13**

(51) Int Cl.:
*H04R 25/00* (2006.01)    *A61B 5/12* (2006.01)

(21) Application number: **19198633.0**

(22) Date of filing: **20.09.2019**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **20.09.2018 EP 18195739**

(71) Applicant: **Oticon A/S**
**2765 Smørum (DK)**

(72) Inventors:
• **LOZA, Agata**
**2765 Smørum (DK)**
• **LUKASZEWSKI, Filip**
**2765 Smørum (DK)**
• **KOSSOWSKI, Grzegorz**
**2765 Smørum (DK)**

(74) Representative: **Demant**
**Demant A/S**
**Kongebakken 9**
**2765 Smørum (DK)**

(54) **HEARING LOSS CLASS CALCULATION**

(57) The present disclosure proves a system, device, computer program and method for classifying hearing loss based on audiogram data, as well as method and system for programming a hearing aid based on such data and classification.

FIG. 1

**Description**

**FIELD**

The present disclosure relates to the field of audiometry, hearing loss, hearing loss compensation and audiograms. More specifically, the disclosure relates to a method for determining a hearing loss class based on audiogram data, and to a system comprising a hearing aid configured for compensating a hearing loss accordingly.

**BACKGROUND**

Studies have shown that audiograms are not easy to understand for non-professionals, especially not for a patient under stress due to just having received a hearing loss diagnosis. Thus, communicating the severity of a hearing loss to a patient in an easy understandable way is complicated.
Algorithms for calculating hearing loss as one single value based on audiogram data are known, but such solutions suffer from the problem that they do not correctly reflect significant hearing loss at only one or few audiogram frequencies.

**SUMMARY**

According to the above, it may be seen as an object of the present disclosure to provide a solution to the problem of providing a simple to understand result of audiogram data, and still giving a meaningful expression of severity of hearing loss.
In a first aspect, the disclosure provides a computer implemented method for classifying hearing loss based on audiogram data, the method comprising

- receiving audiogram data for an ear of a subject,
- determining a value indicative of maximum hearing loss at any available audio frequency, in response to audiogram data for more than four audio frequencies,
- determining a corrected set of hearing loss values for more than four audio frequencies in response to said audiogram data according to a correction algorithm which involves said maximum hearing loss at any available audio frequency, so as to compensate said audiogram data for possible severe hearing loss outlier data,
- calculating a single value indicative of hearing loss according to a predetermined averaging algorithm in response to said corrected set of hearing loss values for more than four audio frequencies,
- determining a hearing loss class from a predetermined list of hearing loss classes in response to the calculated single value, and
- generating an output indicative of the hearing loss class.

Such method is advantageous, since it results in a hearing aid class based on normal audiogram data obtained for an ear of a subject, preferably at all 6 audiogram frequencies: 250 Hz, 500 Hz, 1 kHz, 2 kHz, 4 kHz, and 8 kHz. In spite of expressing hearing loss with one single value indicative of a hearing loss class, the method is capable of reflecting audiogram data in a suitable way also taking into account single frequency hearing losses, i.e. "outliers". In case of audiogram data without any "outliers", the method will provide similar results as other hearing loss class calculation algorithms.
The method is easy to implement on a computer, tablet or other processor device, and it provides a suitable tool for communicating severity of a hearing loss to a patient. E.g. the method can be used either, for a local test and programming or for a remote test and programming of a hearing aid accordingly.
In the following, further features will be described.
Preferably, the single hearing loss value is calculated in response to a predetermined averaging algorithm in response to audiogram data at least at six audio frequencies: 250 Hz, 500 Hz, 1 kHz, 2 kHz, 4 kHz, and 8 kHz.
The averaging algorithm may involve calculating an average value in response to said corrected set of hearing loss values, and further determining said single hearing loss value according to a compensation algorithm involving said value indicative of maximum hearing loss at any available audio frequency.
The single hearing loss value may especially be indicative of a hearing loss percentage value.
The correction algorithm preferably involves determining, for each frequency, a corrected hearing loss value as a maximum value between 1) audiogram data in the form of hearing loss at the frequency, and 2) said maximum hearing loss at any available audio frequency minus a positive value. Especially, the positive value can be selected to be such as 5 dB to 40 dB, such as 10 dB to 30 dB, such as 15 dB to 25 dB, for example such as 20 dB.
The method preferably comprises determining first and second constant values, minLevel and maxLevel, independent of the audiogram data. These constants indicate 0% and 100% hearing level, respectively. Especially, these constants may be set to: minLevel being within 0 dB and 20 dB, such as 10 dB, and maxLevel within 80 dB to 100 dB, such as 90 dB. Specifically, the averaging algorithm may be configured to calculate the single hearing loss value in response to all of: said first and second constant values minLevel, maxLevel, and an average value calculated in response to said audiogram data. Specifically, said average value calculated in response to said audiogram data may be or include an average value calculated in response to said corrected set of hearing loss values, e.g. as a linear average value of the corrected hearing loss values in dB.

The predetermined list of hearing loss classes may comprise at least three disjoint classes, such as 5 disjoint hearing loss classes, e.g. the following 5 classes: "normal hearing", "mild hearing loss", "moderate hearing loss", "severe hearing loss", and "profound hearing loss".

It is to be understood that the method can be performed for one ear or for both ears of a subject.

The step of generating an output indicative of the determined hearing loss class may comprise a visual and/or acoustic presentation of the hearing loss class to the subject.

In a second aspect, the disclosure provides a device comprising a processor programmed to operate according to the method according to the first aspect. The device may be such as a computer, a tablet, a smartphone, or a dedicated device e.g. an audiometry device.

In a third aspect, the disclosure provides a software product comprising executable program code which, when executed on a processor or computer device, causes the processor or computer device to perform the method according to the first aspect.

In a fourth aspect, the disclosure provides a system comprising a hearing aid and a computer, wherein the computer is configured to perform the method according to the first aspect, and wherein the hearing aid is configured to be operated for hearing loss compensation based on the output indicative of the hearing loss class. This may either be in a local setting or in a remote setting, e.g. in the subject's own home, e.g. assisted by a hearing care professional or other trained person via an optional video link. The hearing aid is configured to receive instructions from the computer so as to be adapted to perform hearing loss processing based on these settings.

By 'hearing aid' in the present context is understood a device that is adapted to improve or augment the hearing capability of a user by receiving the transmitted output audio signal and generating a corresponding audio signal, possibly modifying the audio signal and providing the possibly modified audio signal as an audible signal to at least one of the user's ears. Such audible signals may be provided in the form of an acoustic signal radiated into the user's outer ear.

The same advantages and embodiments apply to the second, third, and fourth aspect as already described for the first aspect.

## BRIEF DESCRIPTION OF DRAWINGS

The aspects of the disclosure may be best understood from the following detailed description taken in conjunction with the accompanying figures. The figures are schematic and simplified for clarity, and they just show details to improve the understanding of the claims, while other details are left out. Throughout, the same reference numerals are used for identical or corresponding parts. The individual features of each aspect may each be combined with any or all features of the other aspects. These and other aspects, features and/or technical effect will be apparent from and elucidated with reference to the illustrations described hereinafter in which:

FIG. 1 shows a block diagram of a method,
FIG. 2 shows an example of audiogram data and corrected values according to the disclosure, and
FIG. 3 shows a system embodiment.

## DETAILED DESCRIPTION

The detailed description set forth below in connection with the appended drawings is intended as a description of various configurations. The detailed description includes specific details for the purpose of providing a thorough understanding of various concepts. However, it will be apparent to those skilled in the art that these concepts may be practiced without these specific details. Several aspects of the apparatus and methods are described by various blocks, functional units, modules, components, circuits, steps, processes, algorithms, etc. (collectively referred to as "elements"). Depending upon particular application, design constraints or other reasons, these elements may be implemented using electronic hardware, computer program, or any combination thereof.

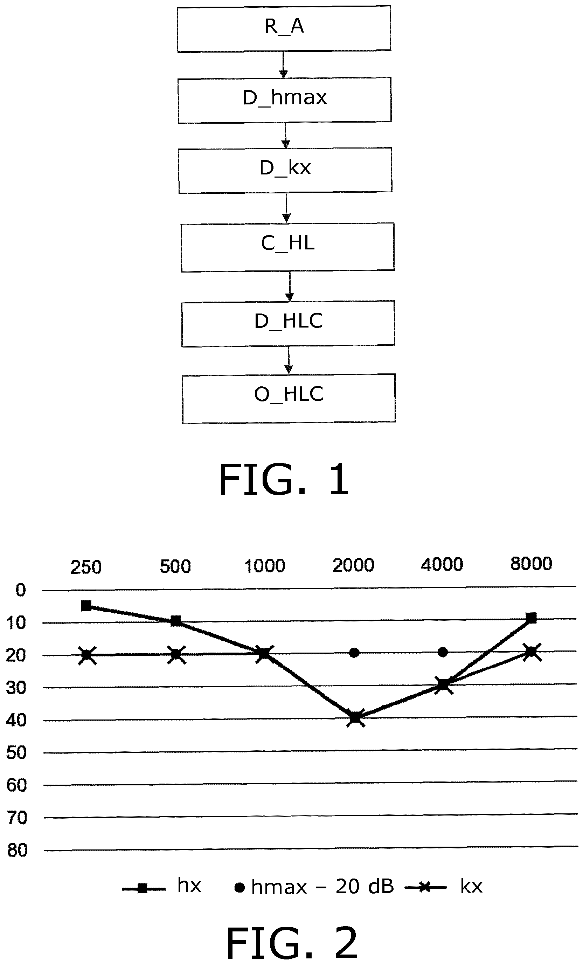

FIG. 1 illustrates a method for classifying hearing loss based on audiogram data, a method which may be performed on a computer or other processor device. The steps are not necessarily to be performed in the exact order described in the following. First step is receiving R_A audiogram data hx for an ear (or both ears) of the subject, e.g. values in a digital format represented as hearing loss values in dB at least at four audio frequencies, most preferably at least the 6 frequencies, such as: 250 Hz, 500 Hz, 1 kHz, 2 kHz, 4 kHz, and 8 kHz. Other frequencies and/or subsets may be used.

Next, determining D_hmax a value indicative of maximum hearing loss hmax at any available audio frequency, in response to audiogram data hx for more than four audio frequencies, i.e.

$$hmax = max[hx] = max[h_{250}, h_{500}, h_{1k}, h_{2k}, h_{4k}, h_{8k}].$$

Next, determining D_kx a corrected set of hearing loss values kx in response to said audiogram data according to a correction algorithm which involves said maximum hearing loss hmax at any available audio frequency, so as to compensate said audiogram data hx for possible severe hearing loss outlier data. This correction algorithm may especially be:

$$kx = max[hx, (hmax - 20 \text{ dB})].$$

In this way, a corrected set of values kx are calculated, where the maximum tolerable difference between the outlier and other frequencies is equal to 20 dB, in this example.

Next, calculating C_HL a single value HL indicative of hearing loss according to an averaging algorithm in response to the corrected hearing loss values kx. Preferably, an average hearing threshold is first calculated as hth = $(k_{250} + k_{500} + k_{1k} + k_{2k} + k_{4k} + k_{8k})/6$, and then HL is calculated as:

$$HL = [(hth - minLevel)/(maxLevel - minLevel)]*100\%,$$

where minLevel and maxLevel defines percentage scale and are independent from input audiogram data. The minLevel defines the 0% and maxLevel defines 100%:

- if hth equals minLevel, then HL is 0%
- if hth equals maxLevel, then HL is 100%

The constants minLevel and maxLevel are to be selected, and especially, it may be preferred to set minLevel = 10 dB and maxLevel = 90 dB.

Next, determining D_HLC a hearing loss class HLC from a predetermined list of hearing loss classes in response to the calculated hearing loss HL. Especially, the hearing loss class HLC can be found based on HL as follows among these 5 disjoint classes: 0-12%: "normal hearing"; 13-38%: "mild hearing loss"; 39-62%: "moderate hearing loss"; 63-90%: "severe hearing loss"; and >90%: "profound hearing loss". The class "normal hearing" could also be defined as <12%, i.e. it is possible that the hearing loss is less than 0%.

Finally, generating O_HLC an output indicative of the hearing loss class HLC. This can be done e.g. as text on a display, either to the subject, or to a professional hearing care person.

With this method, "outliners" will have higher influence on the final result compared to other frequencies. When there is no outliner, then calculated value is similar to other solutions. For example, if a patient in left ear has 40 dB hearing loss for each frequency, but 50 dB at 8000 Hz, the patient will be diagnosed with 39% hearing loss, classified as moderate. Compared with a typical WHO algorithm, it is taken into account 250 Hz and 8000 Hz frequencies, what assures that will more accurately capture hearing loss typical for the elderly, which is more expressed at higher frequencies. Using the algorithm defined above assures that the patient receives an easy to digest information that he can quickly understand and make a decision whether to wear a hearing aid or not. Especially, the proposed algorithm increases chances that non-flat mild/moderate hearing losses will be remedied by a hearing instrument, what will reduce implications for undiagnosed hearing loss due to the socially isolating impact and knock-on effects.

FIG. 2 shows an example of the effect of the above described algorithm, where the audiogram data hx, hmax - 20 dB, and the corrected data kx are illustrated in dB versus frequency in Hz.

With the data in the graph, the above described algorithm will return a HL of 19%, thus corresponding to a HLC with a description "mild hearing loss".

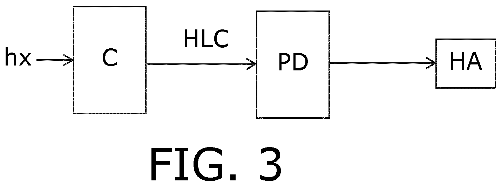

FIG. 3 shows system comprising a computer or tablet C or the like, which is programmed to perform the method for calculating hearing loss class HLC, e.g. as described above, in response to audiogram data hx for a subject. From the computer or tablet C in this system, the obtained hearing loss class HLC is then provided to a programming device PD which in turn is capable of, or configured to, programming a programmable hearing aid HA accordingly, so that the hearing aid will be able to function according to the determined hearing loss class HLC to provide the most suitable hearing loss compensation to assist the subject in hearing.

It is to be understood that such system can be used in various setups, e.g. either in local test and programming or in a remote test and programming. This could be used at a hearing care professional, in a supermarket-type setup or even in the home of the subject. Especially, a videolink may be provided to allow a hearing professional or other trained person in assisting the subject.

As used, the singular forms "a," "an," and "the" are intended to include the plural forms as well (i.e. to have the meaning "at least one"), unless expressly stated otherwise. It will be further understood that the terms "includes," "comprises," "including," and/or "comprising," when used in this specification, specify the presence of stated features, integers, steps, operations, elements, and/or components, but do not preclude the presence or addition of one or more other features, integers, steps, operations, elements, components, and/or groups thereof. It will also be understood that when an element is referred to as being "connected" or "coupled" to another element, it can be directly connected or coupled to the other element but an intervening elements may also be present, unless expressly stated otherwise. Furthermore, "connected" or "coupled" as used herein may include wirelessly connected or coupled. As used herein, the term "and/or" includes any and all combinations of one or more of the associated listed items. The steps of any disclosed method is not limited to the exact order stated herein, unless expressly stated otherwise.

It should be appreciated that reference throughout this specification to "one embodiment" or "an embodiment" or "an aspect" or features included as "may" means that a particular feature, structure or characteristic described in connection with the embodiment is included in at least one embodiment of the disclosure. Furthermore, the particular features, structures or characteristics may be combined as suitable in one or more embodiments of the disclosure. The previous description is provided to enable any person skilled in the art to practice the various aspects described herein. Various modifications to these aspects will be readily apparent to those skilled in the art, and the generic principles defined herein may be applied to other aspects.

The claims are not intended to be limited to the aspects shown herein, but is to be accorded the full scope consistent with the language of the claims, wherein reference to an element in the singular is not intended to mean "one and only one" unless specifically so stated, but rather "one or more." Unless specifically stated otherwise, the term "some" refers to one or more.

Accordingly, the scope should be judged in terms of the claims that follow.

**Claims**

1. A computer implemented method for classifying hearing loss based on audiogram data, the method comprising

   • receiving (R_A) audiogram data (hx) for an ear of a subject,
   • determining (D_hmax), a value indicative of maximum hearing loss (hmax) at any available audio frequency, in response to audiogram data (hx) for more than four audio frequencies,
   • determining (D_kx) a corrected set of hearing loss values (kx) for more than four audio frequencies in response to said audiogram data according to a correction algorithm which involves said maximum hearing loss (hmax) at any available audio frequency, so as to compensate said audiogram data (hx) for possible severe hearing loss outlier data,
   • calculating (C_HL) a single value (HL) indicative of hearing loss according to a predetermined averaging algorithm in response to said corrected set of hearing loss values (kx) for more than four audio frequencies,
   • determining (D_HLC) a hearing loss class (HLC) from a predetermined list of hearing loss classes in response to the calculated single value (HL), and
   • generating (O_HLC) an output indicative of the hearing loss class (HLC).

2. The method according to claim 1, wherein the single value (HL) is calculated in response to a predetermined averaging algorithm in response to audiogram data at least at six audio frequencies.

3. The method according to claim 2, wherein the at least six audio frequencies comprise the frequencies: 250 Hz, 500 Hz, 1 kHz, 2 kHz, 4 kHz, and 8 kHz.

4. The method according to any of the preceding claims, wherein said averaging algorithm involves calculating an average value (hth) in response to said corrected set of hearing loss values (kx), and further determining said single value (HL) according to a compensation algorithm involving said value indicative of maximum hearing loss (hmax) at any available audio frequency.

**5.** The method according to any of the preceding claims, wherein said single value (HL) is indicative of a hearing loss percentage value.

**6.** The method according to any of the preceding claims, wherein said correction algorithm involves determining, for each frequency, a corrected hearing loss value (kx) as a maximum value between 1) audiogram data (hx) in the form of hearing loss at the frequency, and 2) said maximum hearing loss (hmax) at any available audio frequency minus a positive value.

**7.** The method according to claim 6, wherein the averaging algorithm further involves a first constant value (minLevel) to be selected, and a second constant value (maxLevel) to be selected, wherein the first and second constant values (minLevel, maxLevel) are independent from the audio gram data.

**8.** The method according to claim 7, wherein the averaging algorithm is configured to calculate the single value (HL) in response to all of: said first and second constant values (minLevel, maxLevel), and an average value (hth) calculated in response to said audiogram data (hx).

**9.** The method according to claim 8, wherein said average value (hth) calculated in response to said audiogram data (hx) is an average value (hth) calculated in response to said corrected set of hearing loss values (kx).

**10.** The method according to any of the preceding claims, wherein said predetermined list of hearing loss classes (HLC) comprises at least three disjoint classes.

**11.** The method according to any one of the preceding claims, wherein the method is performed for both ears of the subject.

**12.** Device comprising a processor programmed to operate according to the method according to any of the claims 1-11.

**12.** Software product comprising executable program code which, when executed on a processor or computer device, causes the processor or computer device to perform the method according to any of claims 1-11.

**14.** System comprising a hearing aid and a computer, wherein the computer is configured to perform the method according to any one of claims 1-12, and wherein the hearing aid is configured to be operated for hearing loss compensation based on the output indicative of the hearing loss class.

# FIG. 1

── ■ ── hx    ● hmax – 20 dB ──✖── kx

# FIG. 2

hx → | C | —HLC→ | PD | → | HA |

# FIG. 3

Europäisches
Patentamt

European
Patent Office

Office européen
des brevets

# EUROPEAN SEARCH REPORT

Application Number

EP 19 19 8633

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2016/166181 A1 (SHENNIB ADNAN [US]) 16 June 2016 (2016-06-16) * paragraphs [0029], [0031], [0033], [0034], [0047]; figures 1,6 * | 1-14 | INV. H04R25/00 A61B5/12 |
| A | Wikipedia: "Outlier", , 29 April 2018 (2018-04-29), XP055560910, Retrieved from the Internet: URL:https://en.wikipedia.org/w/index.php?title=Outlier&oldid=838787716#cite_note-22 [retrieved on 2019-02-22] * section "Exclusion"; page 5 * | 1-14 | |
| A | US 2008/221719 A1 (MARGOLIS ROBERT H [US] ET AL) 11 September 2008 (2008-09-11) * paragraphs [0013], [0065] - [0066], [0089] - [0091]; figure 1 * | 1-14 | |

TECHNICAL FIELDS
SEARCHED      (IPC)

H04R
A61B

The present search report has been drawn up for all claims

| Place of search | Date of completion of the search | Examiner |
|---|---|---|
| Munich | 6 November 2019 | Rogala, Tomasz |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 19 19 8633

This annex lists the patent family members relating to the patent documents cited in the above-mentioned European search report.
The members are as contained in the European Patent Office EDP file on
The European Patent Office is in no way liable for these particulars which are merely given for the purpose of information.

06-11-2019

| Patent document cited in search report | Publication date | Patent family member(s) | Publication date |
|---|---|---|---|
| US 2016166181 A1 | 16-06-2016 | NONE | |
| US 2008221719 A1 | 11-09-2008 | US 2008221719 A1 | 11-09-2008 |
| | | WO 2008109491 A1 | 12-09-2008 |

EPO FORM P0459

For more details about this annex : see Official Journal of the European Patent Office, No. 12/82